# EUROPEAN PATENT APPLICATION

(11) **EP 1 262 153 A1**
(43) Date of publication of application: **04.12.2002**
(21) Application number: 01810530.4
(22) Date of filing: 31.05.2001
(51) Int. Cl.: A61F 2/06

(54) **Stent for a vascular vessel**

(71) Applicant: Centrafid S.A., 6830 Chiasso (CH)
(72) Inventor: Repetto, Sergio Vincenzo, Carnago (VA) (IT)
(74) Representative: BOVARD AG - Patentanwälte

(57) **Abstract**

The radially expandable stent for holding open a lumen within a body, particularly in a blood vessel, defines a hollow tube structure with an inner and outer wall surface. The outer, the inner, or both wall surfaces are coated with a biologically compatible plastic material or embedded in a biologically compatible plastic material. The hollow tube structure has no attachment means for fastening the biologically compatible plastic material. The plastic material of the inner or outer surface can be impregnated or coated with a pharmaceutical composition for a delivery on site.

## Description

The invention relates to a stent for a vascular vessel such as blood vessels or coronary arteries. Such stents are placed primarily in, and following, balloon angioplasty.

Stents are largely used in treating occluded blood vessels and diseased blood vessels. Stents are placed in more than 1.2 millions procedures per year world-wide. The greatest limitation for stents and their efficacy in treating atherosclerosis lesions in restenosis instant hyperplasia of intimal material and thrombosis in the internal lumen. To combat the 25-35% of instent reocclusion, several novel approaches have been attempted. Stents have been combined with biocompatible coatings, dipped in anticlotting agents, covered in active antibiological pharmaceuticals and even recently have been made radioactive (beta and gamma) in an attempt to prolong stent patency. However, the simplest solution may be mechanical exclusion; a solid-impermeable barrier between the vessel and the inner lumen.

The object of the present invention is to provide stents which are free of the above-mentioned drawback. The stents are impermeable and avoid the direct contact of the metal material of the stent framework with the vascular wall (lesions) of the blood vessel in which the stent is intended to be inserted. Alternatively the stent can be used as an artificial part of a damaged blood vessel.

The present invention provides a novel impermeable stent device having essentially the same expansion properties as known stents. The invention uses existing stent designs comprising a radially expandable framework with interstices in combination with a biologically compatible and safe coating, film or membrane consisting of plastic material which does not affect the mechanical properties of the stent framework. If the stent is expanded e.g. by a balloon, the expanded stent with a plastic coating keeps the new shape.

The subject of the present invention is a radially expandable stent for holding open a lumen within a body, particularly in a blood vessel. The stent defining a hollow tube structure with an inner and outer wall surface is characterised in that the outer, the inner or both wall surfaces are coated with a biologically compatible plastic material or embedded in a biologically compatible plastic material.

Examples of stents according to the present invention are e.g. the following:
- A stent with a membranous barrier (tube or coating material) that covers the interstices of the stent either from inside the lumen, outside or both.
- A stent using an existing stent (balloon mounted) or a stent framework to be treated with a suitable plastic material.

A suitable plastic material for use in the present invention can be a non-permeable expandable plastic membrane including, but not exclusively, polyurethane, polyethylene (low density PE) or silicone plastics e.g. polysiloxane. The thickness of such a membrane is preferably within the range of 0.001 - 0.05 mm.

Since, as a result of proposed manufacturing methods, the plastic material is applied tightly on the hollow tube structure of the stent, no attachment means is required for fastening the biologically compatible plastic material.

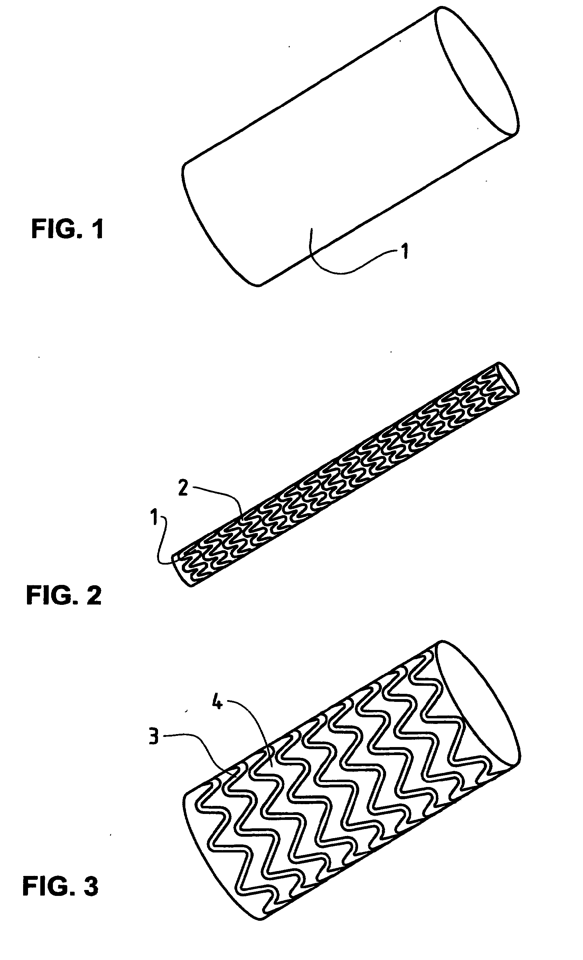

The appended drawings Fig. 1 - 3 illustrate the invention, for better understanding. They show a shrink wrapping tube, an unexpanded stent sheathed with a heat shrinking foil or shrink film according to the invention and the same stent with a foil in an expanded state.

Examples of biocompatible plastics are, for instance, polyurethane and polyethylene materials which have been used in short- and long-term medical applications, i.e. catheters, enteric feeding tubes, permanent pacemaker lead coverings, etc. The application of the plastic membrane therefore to a stent can readily be done using methods known per se. Plasticizing methods can be accomplished in several ways, and examples are indicated below.
a) Dipping: A stent suspended vertically on a mandril can be dipped into a solution of PE or PU, and then air dried, accomplishing a thin membrane over the stent.
b) Shrink wrapping: Sections of extruded PB or PU tubing can be placed over or through the stent (or both), and then heated to a temperature that would adhere the membrane to the stent.
c) Sputtering/spray: A thin membrane can be applied to the stent on a rotating mandril by spraying a PB or PU resin over the outside surface.
d) Wrapping: A thin PE or PU membrane (sheet form) can be wrapped over the stent and secured with an adhesive, suture stitch or heat.
e) Injection moulding: Injection of PB or PU directly into a mould containing a constrained stent.

The plastic coating of the stent according to the present invention can form a pharmaceutical delivery system if a suitable plastic material is chosen which is combined with at least one pharmaceutically active compound. In such a system the active compound can be delivered directly to a target site or systemically by diffusion. The pharmaceutical composition can be coated on the interior or exterior surface of the plastic membrane covering the stent formed by one of the above-mentioned methods. Alternatively the plastic material can be impregnated with a pharmaceutical so that it can be delivered on site in the necessary concentration and time.

Fig. 1 shows a shrink wrapping tube 1 ready for use.

Fig. 2 shows an unexpanded stent wrapped with a shrink wrapping tube. The framework 2 of the stent is in contact with the plastic tubing.

Fig. 3 shows the same combination of an expanded stent framework 3 with an expanded plastic tubing 4.

### Example

A commercially available cardiovascular stent is laminated on its exterior surface with a thin plastic foil, wherein the foil is made of a thermoplastic polymer such as low density polyethylene.

The lamination is achieved by shrink wrapping, a technique in which the strains in a plastic tube are released by raising the temperature of the tube, thus causing it to shrink over the stent. By choosing adequate dimensions and shrinking characteristics of the tube, it is tightly laminated with the stent upon local heating by a fan. (The shrink characteristics of the tube can be adjusted during its manufacture by stretching it under controlled temperatures to produce an orientation of the macromolecules. Upon cooling, the tube retains its stretched condition, but it reverts toward its original dimensions when it is heated.)

The obtained laminated stent is subsequently placed in the graft by means of a catheter according to current practice. Any dislocation of the plastic foil in this procedure is efficiently prevented by the close contact of the foil to the stent. At the position of the vascular closure, the laminated stent is expanded in the graft without rupture of the foil.

## Claims

1. A radially expandable stent for holding open a lumen within a body, particularly in a blood vessel, the stent defining a hollow tube structure with an inner and outer wall surface **characterised in that** the outer, the inner or both wall surfaces are coated with a biologically compatible plastic material or embedded in a biologically compatible plastic material, and the hollow tube structure has no attachment means for the biologically compatible plastic material.

2. The stent of claim 1, wherein the outer wall surface is coated with at least one film or membrane of polyurethane (PU), polyethylene (PE), polypropylene (PP), polybutene (PB) or polysiloxane.

3. The stent of claim 1 or 2, wherein the hollow tube structure consists of an open framework with interstices being embedded in the biologically compatible plastic material.

4. The stent of claim 1 or 2, wherein the hollow tube structure is sheathed with a tubing from biologically compatible plastic material.

5. The stent of one of the claims 1 to 4, wherein the inner or outer wall surface of the stent formed of the biologically compatible plastic material, e.g. polyethylene or polyurethane, has a function of a substrate, and is impregnated or coated with at least one pharmaceutically active compound, e.g. a compound which can be delivered directly to a target site, or which can be delivered systemically by the substrate by diffusion.

6. A method for manufacturing a radially expandable stent for holding open a lumen within a body, particularly in a blood vessel, the stent defining a hollow tube structure with an inner and outer wall surface wherein both wall surfaces are coated with a biologically compatible plastic material **characterised in that** a stent consisting of an open framework with interstices is dipped in a solution of plastic material.

7. A method for manufacturing a radially expandable stent for holding open a lumen within a body, particularly in a blood vessel, the stent defining a hollow tube structure with an inner and outer wall surface wherein both wall surfaces are coated with a biologically compatible plastic material **characterised in that** PB or PU is injected into a mould containing a constrained stent consisting of an open framework with interstices.

8. A method for manufacturing a radially expandable stent for holding open a lumen within a body, particularly in a blood vessel, the stent defining a hollow tube structure with an inner and outer wall surface wherein the outer wall surface is coated with a biologically compatible plastic material **characterised in that** a stent consisting of an open framework with interstices is placed in a tubing of extruded PB or PU, and subsequently the stent with the tubing is exposed to heat.

9. A method for manufacturing a radially expandable stent for holding open a lumen within a body, particularly in a blood vessel, the stent defining a hollow tube structure with an inner and outer wall surface wherein the outer wall surface is coated with a biologically compatible plastic material **characterised in that** a stent consisting of an open framework with interstices is coated by spraying a PB or PU resin over the outer surface.

10. A method for manufacturing a radially expandable stent for holding open a lumen within a body, particularly in a blood vessel, the stent defining a hollow tube structure with an inner and outer wall surface wherein the outer wall surface is coated with a biologically compatible plastic material **characterised in that** a stent consisting of an open framework with interstices is wrapped with a membrane or film of biologically compatible material over the outer surface and the material is secured with an adhesive, suture stitch or heat.
